# EUROPEAN PATENT SPECIFICATION

(11) **EP 4 262 559 B1**
(45) Date of publication and mention of the grant of the patent: **10.07.2024**
(21) Application number: 21823599.2
(22) Date of filing: 09.12.2021
(51) Int. Cl.: A61B 5/1477

(54) **DETERMINING SWEAT PARAMETERS**
BESTIMMUNG VON SCHWEISSPARAMETERN
DÉTERMINATION DES PARAMÈTRES DE SUEUR

(30) Priority: 16.12.2020 EP 20214477
(43) Date of publication of application: 25.10.2023
(73) Proprietor: Koninklijke Philips N.V., 5656 AG Eindhoven (NL)
(72) Inventor: JOHNSON, Mark, Thomas, 5656 AG Eindhoven (NL); VAN LIESHOUT, Ron, Martinus, Laurentius, 5656 AG Eindhoven (NL); GERHARDT, Lutz, Christian, 5656 AG Eindhoven (NL); PELSSERS, Eduard, Gerard, Marie, 5656 AG Eindhoven (NL)
(74) Representative: Philips Intellectual Property & Standards
(86) International application number: PCT/EP2021/084886
(87) International publication number: WO 2022/128711

(56) References cited:
- EP-A1- 3 649 941
- US-A1- 2009 182 216
- US-A1- 2020 155 064

## Description

### FIELD OF THE INVENTION

The invention relates to a device and method for determining sweat parameters of a user.

### BACKGROUND OF THE INVENTION

Non-invasive, semi-continuous and prolonged monitoring of biomarkers that indicate health and well-being is in demand. Such biomarker monitoring may for example find utility in the assessment of dehydration, stress, sleep, children's health and in perioperative monitoring. Sweat is a non-obtrusively accessible bio-fluid and is a rich source of information relating to the physiology and metabolism of the subject.

Some examples of clinically relevant biomarkers in sweat are Na⁺, Cl⁻ or K⁺ ions to monitor dehydration and for cystic fibrosis diagnosis, lactate as an early warning for inflammation (which is relevant to sepsis), glucose for diabetics and neonates, urea for skin conditions and dehydration monitoring, ethanol for alcohol use monitoring, ammonium for metabolic disorders and cortisol for stress monitoring.

The development of reliable sweat sensing has been hampered by several issues, in spite of clinical work showing promising results as early as the 1940s and 1950s. To date the impactful application of sweat analysis has been limited mainly to cystic fibrosis diagnostics, and drugs and alcohol abuse testing.

As summarized by Mena-Bravo and de Castro in "Sweat: A sample with limited present applications and promising future in metabolomics" J. Pharm. Biomed. Anal. 90, 139-147 (2014), it has been found that the results from sweat sensing can be highly variable, and a correlation between values determined from blood and sweat samples appears to be lacking for various biomarkers.

Efforts have been made to address these issues by bringing wearable sensors into nearly immediate contact with sweat as it emerges from the skin. An example is the wearable patch presented by Gao et al. in "Fully integrated wearable sensor arrays for multiplexed in situ perspiration analysis" Nature 529, 509-514 (2016). The patch includes a sensor array for measuring Na+, K+, glucose, lactate, and skin temperature. However, the focus of this study is on the development and integration of the sensors themselves which, whilst evidently crucial, does not address issues relating to sweat collection. The latter is mostly done by placing an absorbent pad with an area in the order of several cm², between the skin and the sensor. The assumption is that, providing ample sweat is produced (hence, tests are done on individuals engaged in intense exercise), the pad will absorb the sweat for analysis, and newly generated sweat will refill the pad and 'rinse away' the old sweat. It is, however, likely that the time-dependent response of the sensor does not directly reflect the actual level of biomarkers over time because of accumulation effects, therefore, continuous reliable sensing over a long period of time is difficult. Such patches may also not be designed to handle the tiny amounts of sweat that are normally produced for individuals at rest and in thermal comfort, e.g. in the in the range of 0.03 - 0.1 nL/min/gland.

EP3649941A1 relates to a device, system and method for estimating an analyte concentration in sweat as released by a sweat gland. It further relates to a switching sensor device for sensing an analyte concentration in sweat as released by a sweat gland and to a trigger sensor device for sensing an analyte concentration in sweat as released by a sweat gland.

There is currently considerable attention towards continuous sweat measurements. One major issue, which needs to be resolved, is that the physiological mechanisms for temperature regulation cause the fast evaporation of small sweat droplets on the skin.

### SUMMARY OF THE INVENTION

The invention is defined by the independent claims. Advantageous embodiments are defined in the dependent claims.

It would be advantageous if a device for determining sweat parameters of a user comprises features reducing unwanted evaporation of sweat. To better address one or more of these concerns, in a first aspect of the invention there is provided a device for determining sweat parameters of a user according to claim 1.

The device is configured to collect sweat from a specific skin area of a subject, i.e. the first skin area, and to use a sensor for determining sweat parameters of the subject from the collected sweat from the said first skin area.

The second area may be a second skin area. Any other skin area of the subject, which does not include the first skin area, is a second skin area and it may be adjacent to the first skin area. The first skin area is defined as the skin area in contact with the collection chamber of the device. Any other skin area not in contact with the collection chamber is a second skin area. Fluid, i.e. sweat or trans-epidermal water (TEW), is excreted in the second skin area. The device is configured to utilize this sweat or TEW, as a source of humidity to moisten its microfluidic structure, increase the vapor pressure within its microfluidic structure and hence reduce evaporation of sweat used for determining sweat parameters, i.e. sweat originating from the first skin area.

By decreasing evaporation of sweat from the first skin area, the available quantity of liquid sweat that reaches the sensor is increased and the accumulation of dried components from sweat, hindering reliable and reproducible flow within the device may be prevented. Furthermore, sweat evaporation may change the concentration of biomarkers in the liquid sweat reaching the sensor, due to the decrease of total liquid sweat volume. Additionally, sweat rate determination may also be hindered by sweat evaporation. Therefore, decreasing sweat evaporation may also improve the outcome of the measurements of the sweat sensor.

The device may be a wearable device. A wearable device may improve ease of use, be comfortable for the subject to wear for prolonged periods and is suitable for continuous measurements for prolonged periods, e.g. days or weeks.

The fluid from the second skin area, i.e. sweat or TEW to be used for moistening the microfluidic structure of the device, may be in liquid or vapor state, depending on the embodiment of the invention. For example, the said fluid may be evaporated in the evaporation control chamber, in order to increase the humidity within the microfluidic structure, which is connected, i.e. fluidically connected, to the evaporation control chamber. In another example, the said fluid is collected in the evaporation control chamber and transported to the microfluidic structure in liquid state, to wet the sensor or other components of the microfluidic structure and maintain a humid environment within the device.

Sweat from the first skin area is collected in the collection chamber. The collection chamber may comprise conically shaped pillars. The conically shaped pillars reduce the total volume and the impact of fluidic movement in the collection chamber, thereby decreasing the time of filling the collection chamber. The collection chamber may comprise a hydrogel or a wicking material.

Furthermore, the microfluidic structure comprises a first absorber, which is connected via a microfluidic channel with the collection chamber and the sensor. Sweat from the first skin area is collected in the collection chamber, and due to the pressure of the sweat glands and capillary action, it is transported along the sensor and finally into the first absorber. Alternatively, sweat may be moved along the microfluidic channel based on discretized droplet transport, e.g. based on passive chemical gradient or electrowetting techniques. As the sweat received by the first absorber has already passed the sensor, it is no longer needed for measurements and it can be stored and evaporated. The moistening of the first absorber keeps the microfluidic channel moistened, thus it helps to reduce evaporation of sweat of the first skin area, which has not yet passed the sensor. The gradual evaporation of sweat from the first absorber also helps to create a pressure gradient within the microfluidic channel, thus, helping the transportation of sweat from the collection chamber towards the sensor and finally into the absorber.

The moistening of the microfluidic structure from the evaporation control chamber, results in the decrease of the evaporation rate of sweat from the first absorber. Although evaporation of sweat from the first absorber is desired, it is also desired that this evaporation rate should be limited, because fast evaporation of sweat from the first absorber would also lead to fast evaporation of sweat within the microfluidic channel, thus, evaporation of sweat before it reaches the sensor.

Furthermore, the sensor comprises an exhaust for venting sweat that has entered the sensor. The exhaust removes sweat, such that it does not pile inside the sensor and thus, room is reserved for new sweat to enter and to be measured by the sensor. For the same reason as with the first absorber, although removal of sweat from the sensor though the exhaust is desired, it is also desired that the removal rate should be limited.

The evaporation control chamber is directly in connection with the first absorber or the exhaust of the sensor of the microfluidic structure. The fluid of the second skin area, in vapor, liquid or both states may directly flow towards the first absorber, or to the exhaust of the sensor, or to both the first absorber and the exhaust of the sensor. Directly moistening the first absorber or the exhaust of the sensor, being components connected to the microfluidic channel, decreases evaporation of sweat within the microfluidic channel, through which sweat from the first skin area moves to reach the sensor.

In another example, the evaporation control chamber may comprise a second absorber, which is configured to receive fluid collected from the second skin area and is configured to moisten the microfluidic structure. The evaporation control chamber may also comprise a fluid transporter, configured to transport droplets of fluid to the second absorber.

The fluid transporter e.g. a transporter moving discretized droplets based on passive chemical gradient or electrowetting, provides faster transport of fluid than capillary action. The advantage of a second absorber is that it is configured for stimulating evaporation of fluid as fast as possible, leading to an increased rate of moistening of the microfluidic structure.

In a further example, the evaporation control chamber may comprise a fluid transporter, configured to transport droplets of fluid to the first absorber.

In another example, the evaporation control chamber may also comprise heaters. The skin temperature may be sufficient to create sufficient evaporation in the evaporation control chamber. However, it may be beneficial to stimulate evaporation of fluid originating from the second skin area, from the thermal energy produced by heaters.

Furthermore, the device comprises a vent configured to release vapors out of the device and a condensation element in connection to the vent and the microfluidic structure and configured to condensate vapors. Furthermore, the device is constructed with a first material at the collection chamber and the sensor, having a first thermal resistance and a second material at the vent and the condensation element, having a second thermal resistance. Moreover, the first thermal resistance is configured to be higher than the second thermal resistance, to condensate vapors before being released out of the device.

The vent releases vapors out of the device, to reduce pressure within the device and allow new sweat from the first skin area to be collected and transported to the sensor. In order to prevent fast evaporation of sweat from the first skin area within the microfluidic structure, and therefore within the microfluidic channel, humidity should remain high. Therefore, a temperature gradient is created to facilitate condensation of vapors before exiting the device through the vent. To create the temperature gradient, the device is constructed with a first material, which is used for the part of the device encompassing the collection chamber and the sensor. The first material has a first thermal resistance. The device is constructed with second material at the part, which encompasses the vent and the condensation element. The second material has a second thermal resistance, and the first thermal resistance is higher than the second thermal resistance.

The first material having greater thermal resistance than the material used at the vent and the condensation element creates a temperature gradient, with higher temperature, close to the skin, i.e. at the collection chamber, than the temperature at the vent and the condensation element. The lower thermal resistance of the second material at the vent and the condensation element, results to them having a temperature closer to the environmental temperature, which is colder than the vapors. The contact of the vapors with the colder surface will result in condensation of part of the vapors. Therefore, the condensed vapors will flow back to the device and reduce the loss of humidity from within the device.

In an example, the second area is a surface of the condensation element. In this example, the device does not need to collect fluid from a second skin area. Sweat from the first skin area that has passed the sensor, in the form of vapors, increases humidity inside the microfluidic structure. These vapors are condensed at the surface of the condensation element, flowing back to the device and reducing the loss of humidity from within the device.

The device may further comprise passive cooling elements such as cooling blocks or active cooling elements, such as Peltier elements, to increase the cooling of the vent and the condensation element and therefore, to increase condensation of vapors before exiting the device.

The invention also provides a method for determining sweat parameters of a user according to claim 7.

The present method aims to increase the available sweat for the sensor to determine a sweat parameter, by increasing the humidity inside the device comprising the sensor and therefore, decreasing the evaporation of sweat.

These and other aspects of the invention will be apparent from and elucidated with reference to the embodiments described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figs. 1a and 1b show a first example of the device not according to the invention.
Fig. 2 shows a second example of the device according to the invention.
Fig. 3 shows a third example of the device according to the invention.
Fig. 4 shows a fourth example of the device according to the invention.
Figs. 5a and 5b show a fifth example of the device according to the invention.
Fig. 6 shows a flow diagram for an example of the method according to the invention.

### DESCRIPTION OF EMBODIMENTS

The invention will be described with reference to the Figures. The detailed description and specific examples, while indicating exemplary embodiments of the devices and methods, are intended for purposes of illustration only and are not intended to limit the scope of the invention, which is defined in the appended claims. The Figures are merely schematic and are not drawn to scale. It should also be understood that the same reference numerals are used throughout the Figures to indicate the same or similar parts.

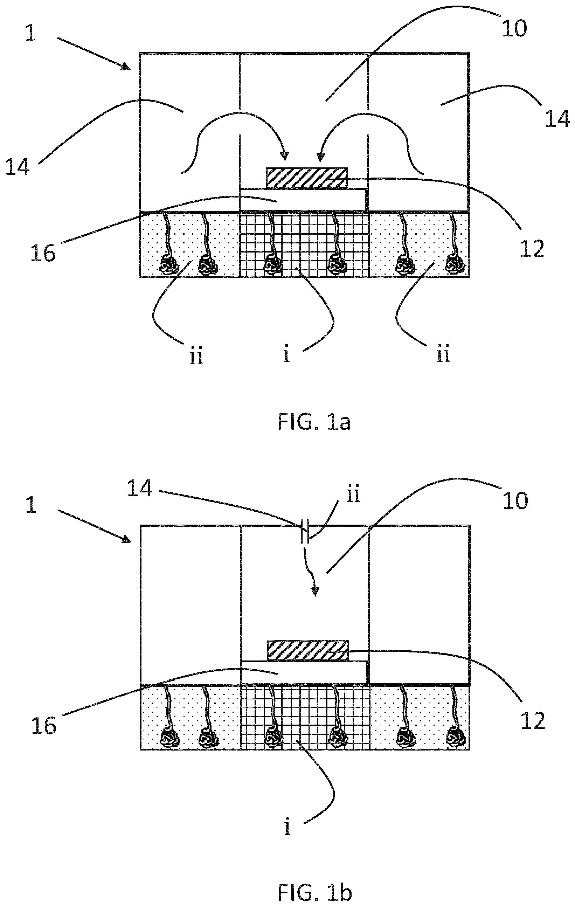

FIGs. 1a and 1b show the device 1 which comprises a microfluidic structure 10 having a collection chamber 16 where sweat from a first skin area i is collected, and a sensor 12 for determining a sweat parameter from the first skin area i. The device 1 also comprises an evaporation control chamber 14, which is connected to the microfluidic structure 10 and collects fluid from a second area ii. In FIG. 1a the second area ii is a second skin area. In FIG. 1b the second area is an area within the device.

Evaporation of sweat from the first skin area i can limit the availability of sweat that is needed for the sensor 12. The humidity in the microfluidic structure 10 is controlled by the evaporation control chamber 14 keeping the humidity relatively high so that only a small part of sweat from the first skin area i as collected in the collection chamber 16 will evaporate.

The collected fluid from the second area may be in liquid or vapor state. It can directly flow towards the microfluidic structure 10 or for example, in case of vapors, it would fill the space within the device and as the evaporation control chamber 14 is connected to the microfluidic structure 10, it would also fill the space of the microfluidic structure 10. Thus, a humid environment is maintained within the microfluidic structure 10 and evaporation of sweat originating from the first skin area i, which is used for determining sweat parameters, is reduced. The humid environment in the microfluidic structure may for example have relative humidity (RH) above the typical RH values in an indoor environment and may for example be 80-90%.

The sweat parameter determined by the sensor 12 is a concentration of a biomarker in sweat and optionally sweat rate. Sensors for determining concentration of biomarkers can, for example work based on electroanalytical techniques (e.g. chronoamperometry, potentiometry, voltammetry, electrochemical impedance spectroscopy, piezo-electricity) or optical techniques (e.g. colorimetry, fluorometry). Examples of sensors that are capable of sensing a parameter indicative of the sweat rate of a person may for example be skin response (GSR) sensors, calorimetric flow sensors, microbalances or resonance-based sensors.

Although not shown in FIGs. 1a and 1b, the device 1 may comprise an output e.g. a display, for communicating the determined sweat parameters to a user and/or a communications component to connect with an external output device for communicating the determined sweat parameters to a user.

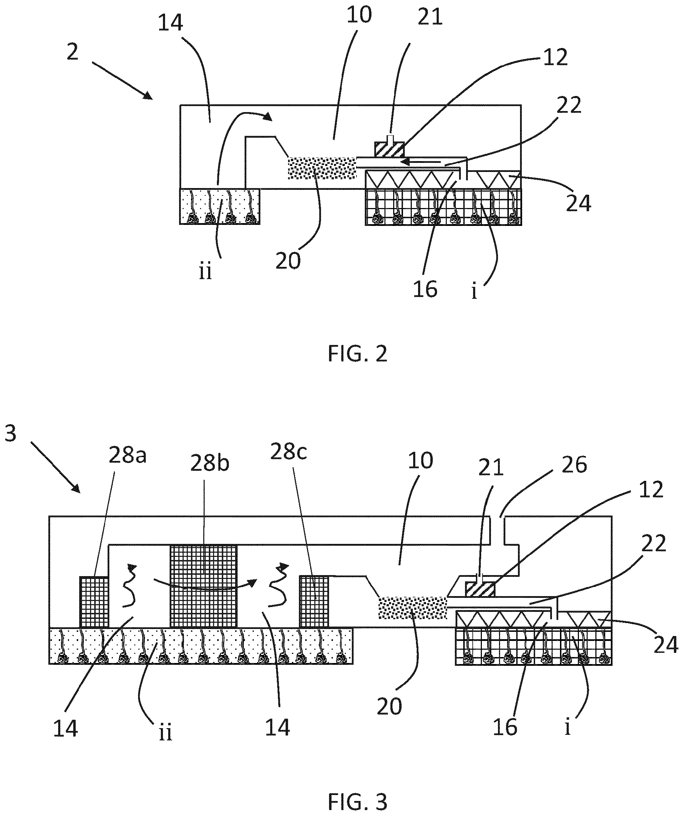

FIG. 2 shows an exemplary embodiment of the invention. A device 2 comprises a microfluidic structure 10 and an evaporation control chamber 14. The microfluidic structure 10 comprises a collection chamber 16 and a sensor 12 connected via a microfluidic channel 22.

The device 2 comprises a first absorber 20, which is connected to the microfluidic channel and receives sweat from the first skin area i, which has passed the sensor 12.

Optionally, the sensor 12 comprises an exhaust 21 configured to vent sweat out of the sensor 12 and into the microfluidic structure 10.

Optionally, the collection chamber 16 comprises conically shaped pillars 24. The conically shaped pillars 24 are configured such that they do not block sweat from moving within the collection chamber 16 and towards the sensor 12.

In FIG. 2, sweat from a first skin area i is collected in the collection chamber 16, from the sweat glands ejecting onto the skin. The pressure of the sweat glands aided by the capillary action of the microfluidic channel 22 transports the collected sweat from the first skin area i through the microfluidic channel 22 towards the sensor 12 and finally into the first absorber 20. When passing the sensor 12, the sensor measures a sweat parameter. Fluid from the second skin area ii is collected in the evaporation control chamber 14, which is used to moisten the microfluidic structure 10.

In a further example, the collected fluid is connected directly to the first absorber 20 or to the optional exhaust 21 of the sensor 12. This direct moistening of the first absorber 20 or the exhaust 21 of the sensor 12 may quicken the decrease of evaporation of sweat within the microfluidic channel 22, through which sweat from the first skin area i moves to reach the sensor 12.

FIG. 3 shows another exemplary embodiment of the invention. Similarly to FIGs. 1 and 2, the device 3 comprises a microfluidic structure 10 and an evaporation control chamber 14, in connection to the microfluidic structure 10. The microfluidic structure 10 comprises a collection chamber 16, optionally comprising conically shaped pillars 24, a sensor 12 optionally having an exhaust 21 and a first absorber 20, connected via a microfluidic channel 22. In FIG. 3, the evaporation control chamber 14 may comprise heaters 28a, 28b or 28c. The device 3 also comprises a vent 26. The optional heater 28b is configured to allow humidity to pass towards the microfluidic structure 10.

The evaporation control chamber 14 evaporates fluid, namely sweat or TEW from a second skin area ii, and this local evaporation of fluid creates a humidity, which disperses in the microfluidic structure 10 and thereby minimizes evaporation within the microfluidic structure 10. Excess humidity is exhausted from the device through the vent 26. The vent 26 may comprise a humidity permeable membrane.

The skin temperature may be sufficient to create sufficient evaporation in the evaporation control chamber 14, however it may be beneficial to stimulate the creation of humidity, with the use of the optional heaters 28a, 28b, and/or 28c.

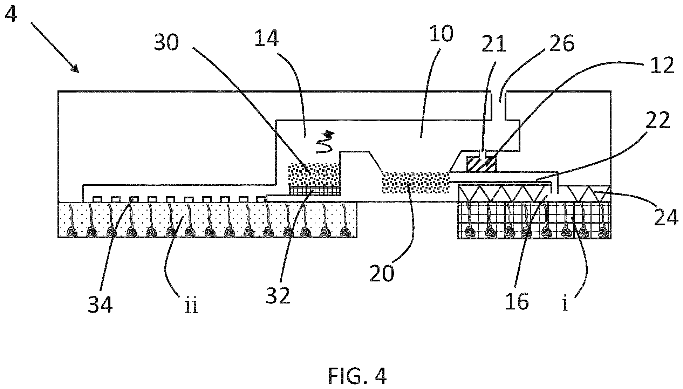

FIG. 4 shows another exemplary embodiment of the invention. In FIG. 4 the microfluidic structure 10 of a device 4 comprises the same features and operates as in the embodiment illustrated in FIG. 3. The evaporation control chamber 14 comprises a fluid transporter 34, a second absorber 30, and optionally a heater 32. The fluid transporter 34 transports sweat and/or TEW originating from the second skin area ii, to the second absorber 30. The sweat and/or TEW, which is accumulated in the second absorber 30, evaporates, optionally aided by the heater 32. The created humidity disperses in the microfluidic structure 10, thereby reducing evaporation within the microfluidic structure 10.

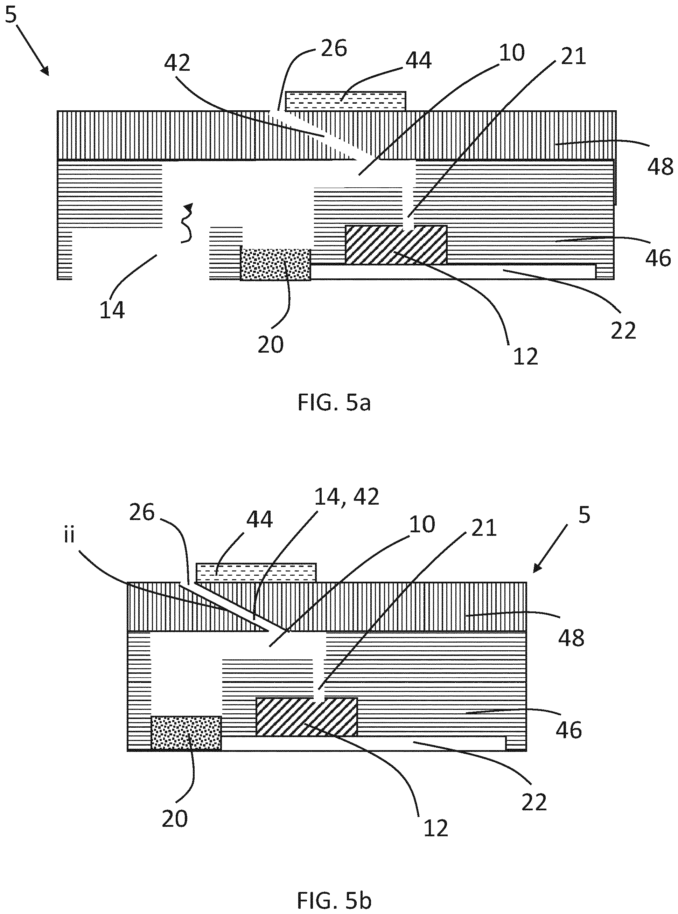

FIG. 5a and 5b show another exemplary embodiment of the invention. A device 5 comprises a microfluidic structure 10 having a sensor 12, which receives sweat from a first skin area i via a microfluidic channel 22. The sensor 12 comprises an exhaust 21, configured to vent sweat out of the sensor 12 and into the microfluidic structure 10. The microfluidic structure 10 comprises a first absorber 20 receiving sweat, which has passed the sensor 12. The device 5 comprises a vent 26, a condensation element 42 and optionally a cooling element 44. The evaporation control chamber 14 is in connection with the microfluidic structure 10. The vent is connected to the microfluidic structure via the condensation element 42.

In FIG. 5b, the second area ii is a surface of the condensation element 42. The evaporation control chamber 14 is the condensation element 42. In further examples, the device may comprise two second areas ii, i.e. a second skin area and a surface of the condensation element 42.

The device 5 is constructed with a first material 46 having a first thermal insulation, and a second material 48 having a second thermal insulation. The first thermal insulation is higher than the second thermal insulation. The first material 46 is used for the part of the device 5, which encompasses the microfluidic channel 22, the sensor 12 and the first absorber 20. The second material 48 is used for the part of the device encompassing the vent 26 of the device, the condensation element 42 and the optional cooling elements 44. Optionally, the first and second materials 46 and 48 are used only in the aforementioned parts of the housing, with the rest of the housing comprising a third material. In the example shown in FIG. 5a, a first material 46 is used for the part of the device 5 encompassing the microfluidic structure 10 and evaporation control chamber 14, and a second material 48 is used for the part of the device 5, where the vent 26, the condensation element 42 and the optional cooling elements 44 are located.

For reducing the evaporation of sweat from the first skin area i within the microfluidic structure 10, the humidity in the microfluidic structure 10 should be high. In order to increase the humidity in the microfluidic structure, in FIG. 5a fluid from the second skin area ii (e.g. sweat and/or trans-epidermal water (TEW)) and in FIG. 5b, sweat from the first skin area i is evaporated and therefore, temperature should be increased. However, the rise of the temperature will also lead to a faster evaporation of sweat from the first absorber 20 and/or from within the sensor 12, escaping from exhaust 21 and finally will vent out from the device through the vent 26.

In order to prevent loss of humidity from the device, leading to loss of liquid across the microfluidic channel 22, through evaporation from the first absorber 20 and/or the exhaust 21, humidity should remain high. Therefore, a temperature gradient is created with direction from the skin surface (higher temperature) towards the vent of the device (lower temperature) to facilitate condensation. The temperature gradient is created by using the first material 46 having higher thermal insulation than the second material 48. Therefore, heat transfer between the device and the external environment is reduced close to the skin, while heat transfer between the device and the environment is higher at the vent 26 and condensation element 42. Thus, temperature of the device close to the skin is higher than temperature of the device close to the vent 26 and condensation element 42. The contact of the vapors with the colder surface of the condensation element 42, will result in condensation of part of the vapors and the condensed vapors will flow back to the device, reducing loss of humidity within the microfluidic structure 10.

The cooling of the vent and the condensation element 42 may be increased by optionally adding passive cooling elements (i.e. cooling blocks) or active cooling elements (e.g. Peltier).

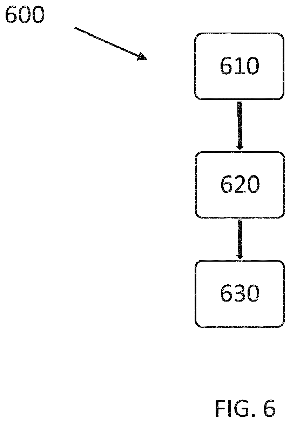

FIG. 6 shows an example of a method 600 for determining sweat parameters of a user. The method comprises the steps of collecting sweat 610 originating from a first skin area i, collecting fluid 620 at a second area ii to be utilized for moistening a microfluidic structure 10, and determining a sweat parameter 630 from sweat originating from the first skin area i. The microfluidic structure 10 comprises a sensor 12, which is used for determining the sweat parameter from sweat originating from the first skin area i.

The determined sweat parameter is a concentration of a biomarker in sweat from the first skin area i and optionally sweat rate.

The second area ii may be a second skin area ii. In another example, the second area is formed by a condensation element configured to condensate vapors released by a device used to implement the method.

The method may further comprise providing an output to the user, with the determined sweat parameter.

In summary, the description describes a device for determining sweat parameters of a user, the device comprising a microfluidic structure 10 having a collection chamber 16 configured to collect sweat from a first skin area i, and a sensor 12 configured to determine a sweat parameter from sweat from the first skin area i. The device also comprises an evaporation control chamber 14, which is connected to the microfluidic structure 10, configured to utilize fluid collected at a second area ii to moisten the microfluidic structure 10. The moistening of the microfluidic structure 10 aims to increase the available sweat for the sensor to determine a sweat parameter, by increasing the humidity inside the microfluidic structure 10 and thus, decreasing the evaporation of sweat. A method 600 for determining sweat parameters of a user is also provided.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. Measures recited in mutually different dependent claims may advantageously be used in combination. The scope of the invention is defined in the appended claims.

## Claims

1. A device (1) for determining sweat parameters of a user, the device comprising:
a microfluidic structure (10) comprising a collection chamber (16) configured to collect sweat from a first skin area (i), and a sensor (12) configured to determine a sweat parameter from sweat from the first skin area (i), wherein the microfluidic structure (10) comprises a first absorber (20), connected via a microfluidic channel (22) with the collection chamber (16) and the sensor (12), wherein the first absorber (20) is configured to receive sweat from the first skin area (i), having passed the sensor (12), and
an evaporation control chamber (14), in fluidic connection with the microfluidic structure (10), configured to utilize fluid collected at a second area (ii) to moisten the microfluidic structure (10), wherein the second area (ii) is
a second skin area, and/or
a surface of a condensation element (42) configured to condensate vapors released by the device (1) through a vent (26) connected to the microfluidic structure via the condensation element (42), and the sensor (12) comprising an exhaust (21) configured to vent sweat out of the sensor (12) and into the microfluidic structure (10), and
wherein the evaporation control chamber (14) is in direct connection with the first absorber (20) and is configured to moisten the first absorber.

2. The device according to claim 1, wherein:
the second area (ii) is a second skin area.

3. The device according to claim 2, wherein:
the evaporation control chamber (14) is in direct connection with the exhaust (21) of the sensor (12) and is configured to moisten the exhaust of the sensor.

4. The device according to any of claims 1-3, wherein:
the second area (ii) is a second skin area, and
the evaporation control chamber (14) comprises a second absorber (30), configured to receive fluid collected from the second skin area (ii) and configured to moisten the microfluidic structure (10), and
a fluid transporter (34), configured to transport droplets of fluid from the second skin area (ii) to the second absorber (30).

5. The device according to any of claims 1-4, wherein:
the evaporation control chamber (14) is configured to evaporate the fluid collected from the second skin area (ii), optionally with the use of a heater (28a, 28b, 28c, 32).

6. The device according to any preceding claims, wherein the device comprises:
the vent (26) configured to release vapors out of the device;
the condensation element (42) in connection to the vent and the microfluidic structure (10) and configured to condensate vapors, wherein the device is constructed with:
a first material (46) at the collection chamber (14) and the sensor (12), the first material (46) having a first thermal resistance;
a second material (48) at the vent (26) and the condensation element (42), the second material (48) having a second thermal resistance, and wherein:
the first thermal resistance is higher than the second thermal resistance to condensate vapors at the condensation element (42) before being released out of the device through the vent (26).

7. A method (500) for determining sweat parameters of a user, the method comprising:
collecting sweat (510) originating from a first skin area (i);
collecting fluid (520) at a second area (ii) to moisten a microfluidic structure (10) of a device (1) having
a sensor (12) configured to determine a sweat parameter from sweat from a first skin area (i), wherein the microfluidic structure (10) comprises a first absorber (20), connected via a microfluidic channel (22) with the collection chamber (16) and the sensor (12), wherein the first absorber (20) is configured to receive sweat from the first skin area (i), having passed the sensor (12), and
an evaporation control chamber (14), in fluidic connection with the microfluidic structure (10), configured to utilize fluid collected at a second area (ii) to moisten the microfluidic structure (10), wherein the second area (ii) is
a second skin area, and/or
a surface of a condensation element (42) configured to condensate vapors released by the device (1) through a vent (26) connected to the microfluidic structure via the condensation element (42), and the sensor (12) comprising an exhaust (21) configured to vent sweat out of the sensor (12) and into the microfluidic structure (10), and
wherein the evaporation control chamber (14) is in direct connection with the first absorber (20) and is configured to moisten the first absorber, and
determining a sweat parameter (530) from sweat originating from the first skin area (i) using the sensor (12).

## Patentansprüche

1. Vorrichtung (1) zum Bestimmen von Schweißparametern eines Benutzers, wobei die Vorrichtung Folgendes umfasst:
eine Mikrofluidstruktur (10) umfassend eine Sammelkammer (16), die konfiguriert ist, Schweiß von einem ersten Hautbereich (i) zu sammeln, und einen Sensor (12), der konfiguriert ist, einen Schweißparameter aus Schweiß von dem ersten Hautbereich (i) zu bestimmen, wobei die Mikrofluidstruktur (10) einen ersten Absorber (20) umfasst, der über einen Mikrofluidkanal (22) mit der Sammelkammer (16) und dem Sensor (12) verbunden ist, wobei der erste Absorber (20) konfiguriert ist, Schweiß von dem ersten Hautbereich (i) aufzunehmen, der den Sensor (12) passiert hat, und
eine Verdunstungskontrollkammer (14), die in Fluidverbindung mit der Mikrofluidstruktur (10) steht und konfiguriert ist, in einem zweiten Bereich (ii) gesammeltes Fluid zu verwenden, um die Mikrofluidstruktur (10) zu befeuchten, wobei der zweite Bereich (ii)
ein zweiter Hautbereich und/oder
eine Oberfläche eines Kondensationselements (42) ist, das konfiguriert ist, von der Vorrichtung (1) freigesetzte Dämpfe durch eine Entlüftung (26), die über das Kondensationselement (42) mit der Mikrofluidstruktur verbunden ist, zu kondensieren, und wobei der Sensor (12) einen Austritt (21) umfasst, der konfiguriert ist, Schweiß aus dem Sensor (12) und in die Mikrofluidstruktur (10) abziehen zu lassen, und
wobei die Verdunstungskontrollkammer (14) in direkter Verbindung mit dem ersten Absorber (20) steht und konfiguriert ist, den ersten Absorber zu befeuchten.

2. Vorrichtung nach Anspruch 1, wobei:
der zweite Bereich (ii) ein zweiter Hautbereich ist.

3. Vorrichtung nach Anspruch 2, wobei:
die Verdunstungskontrollkammer (14) in direkter Verbindung mit dem Austritt (21) des Sensors (12) steht und konfiguriert ist, den Austritt des Sensors zu befeuchten.

4. Vorrichtung nach einem der Ansprüche 1-3, wobei:
der zweite Bereich (ii) ein zweiter Hautbereich ist und
die Verdunstungskontrollkammer (14) einen zweiten Absorber (30) umfasst, der konfiguriert ist, aus dem zweiten Hautbereich (ii) gesammeltes Fluid aufzunehmen, und konfiguriert ist, die Mikrofluidstruktur (10) zu befeuchten, und
einen Fluidtransporter (34), der konfiguriert ist, Fluidtröpfchen von dem zweiten Hautbereich (ii) zu dem zweiten Absorber (30) zu transportieren.

5. Vorrichtung nach einem der Ansprüche 1-4, wobei:
die Verdunstungskontrollkammer (14) konfiguriert ist, das aus dem zweiten Hautbereich (ii) gesammelte Fluid, optional unter Verwendung einer Heizvorrichtung (28a, 28b, 28c, 32), zu verdunsten.

6. Vorrichtung nach einem der vorstehenden Ansprüche, wobei die Vorrichtung Folgendes umfasst:
die Entlüftung (26), die konfiguriert ist, Dämpfe aus dem Gerät freizusetzen;
das Kondensationselement (42) in Verbindung mit der Entlüftung und der Mikrofluidstruktur (10) und konfiguriert, Dämpfe zu kondensieren, wobei die Vorrichtung konstruiert ist aus:
einem ersten Material (46) an der Sammelkammer (14) und dem Sensor (12), wobei das erste Material (46) einen ersten Wärmewiderstand aufweist;
einem zweiten Material (48) an der Entlüftung (26) und dem Kondensationselement (42), wobei das zweite Material (48) einen zweiten Wärmewiderstand aufweist, und wobei:
der erste Wärmewiderstand höher ist als der zweite Wärmewiderstand, um Dämpfe am Kondensationselement (42) zu kondensieren, bevor sie durch die Entlüftung (26) aus der Vorrichtung freigesetzt werden.

7. Verfahren (500) zum Bestimmen von Schweißparametern eines Benutzers, wobei das Verfahren Folgendes umfasst:
Sammeln von Schweiß (510), der aus einem ersten Hautbereich (i) stammt;
Sammeln von Fluid (520) in einem zweiten Bereich (ii), um eine Mikrofluidstruktur (10) einer Vorrichtung (1) zu befeuchten, die Folgendes aufweist:
einen Sensor (12), der konfiguriert ist, einen Schweißparameter aus Schweiß von einem ersten Hautbereich (i) zu bestimmen, wobei die Mikrofluidstruktur (10) einen ersten Absorber (20) umfasst, der über einen Mikrofluidkanal (22) mit der Sammelkammer (16) und dem Sensor (12) verbunden ist, wobei der erste Absorber (20) konfiguriert ist, Schweiß von dem ersten Hautbereich (i) aufzunehmen, der den Sensor (12) passiert hat, und
eine Verdunstungskontrollkammer (14), die in Fluidverbindung mit der Mikrofluidstruktur (10) steht und konfiguriert ist, in einem zweiten Bereich (ii) gesammeltes Fluid zu verwenden, um die Mikrofluidstruktur (10) zu befeuchten, wobei der zweite Bereich (ii)
ein zweiter Hautbereich und/oder
eine Oberfläche eines Kondensationselements (42) ist, das konfiguriert ist, von der Vorrichtung W freigesetzte Dämpfe durch eine Entlüftung (26), die über das Kondensationselement (42) mit der Mikrofluidstruktur verbunden ist, zu kondensieren, und wobei der Sensor (12) einen Austritt (21) umfasst, der konfiguriert ist, Schweiß aus dem Sensor (12) und in die Mikrofluidstruktur (10) abziehen zu lassen, und
wobei die Verdunstungskontrollkammer (14) in direkter Verbindung mit dem ersten Absorber (20) steht und konfiguriert ist, den ersten Absorber zu befeuchten, und
Bestimmen eines Schweißparameters (530) aus Schweiß, der aus dem ersten Hautbereich (i) stammt, unter Verwendung des Sensors (12).

## Revendications

1. Dispositif (1) pour déterminer des paramètres de transpiration d'un utilisateur, le dispositif comprenant :
une structure microfluidique (10) comprenant une chambre de collecte (16) configurée pour collecter de la transpiration provenant d'une première zone de peau (i), et un capteur (12) configuré pour déterminer un paramètre de transpiration à partir de la transpiration provenant de la première zone de peau (i), dans lequel la structure microfluidique (10) comprend un premier absorbeur (20), relié par le biais d'un canal microfluidique (22) à la chambre de collecte (16) et au capteur (12), dans lequel le premier absorbeur (20) est configuré pour recevoir de la transpiration en provenance de la première zone de peau (i), ayant traversé le capteur (12), et
une chambre de régulation de l'évaporation (14), en connexion fluidique avec la structure microfluidique (10), configurée pour utiliser un fluide collecté au niveau d'une seconde zone (ii) pour humidifier la structure microfluidique (10), dans lequel la seconde zone (ii) est
une seconde zone de peau, et/ou
une surface d'un élément de condensation (42) configurée pour condenser des vapeurs libérées par le dispositif (1) par un évent (26) relié à la structure microfluidique par le biais de l'élément de condensation (42), et le capteur (12) comprenant un échappement (21) configuré pour évacuer la transpiration hors du capteur (12) et jusque dans la structure microfluidique (10), et
dans lequel la chambre de régulation d"évaporation (14) est en connexion directe avec le premier absorbeur (20) et est configurée pour humidifier le premier absorbeur.

2. Dispositif selon la revendication 1, dans lequel
la seconde zone (ii) est une seconde zone de peau.

3. Dispositif selon la revendication 2, dans lequel :
la chambre de régulation d"évaporation (14) est en connexion directe avec l'échappement (21) du capteur (12) et est configurée pour humidifier l'échappement du capteur.

4. Dispositif selon l'une quelconques des revendications 1-3, dans lequel :
la seconde zone (ii) est une seconde zone de peau, et
la chambre de régulation de l'évaporation (14) comprend un second absorbeur (30), configuré pour recevoir un fluide collecté à partir de la seconde zone de peau (ii) et configuré pour humidifier la structure microfluidique (10), et
un transporteur de fluide (34), configuré pour transporter des gouttelettes de fluide depuis la seconde zone de peau (ii) jusqu'au second absorbeur (30).

5. Dispositif selon l'une quelconque des revendications 1-4, dans lequel
la chambre de régulation de l'évaporation (14) est configurée pour évaporer le fluide collecté à partir de la seconde zone de peau (ii), facultativement à l'aide d'un dispositif de chauffage (28a, 28b, 28c, 32).

6. Dispositif selon une quelconque revendication précédente, dans lequel le dispositif comprend :
l'évent (26) configuré pour libérer des vapeurs hors du dispositif ;
l'élément de condensation (42) en connexion avec l'évent et la structure microfluidique (10) et configuré pour condenser des vapeurs, dans lequel le dispositif est construit avec :
un premier matériau (46) au niveau de la chambre de collecte (14) et du capteur (12), le premier matériau (46) présentant une première résistance thermique ;
un second matériau (48) au niveau de l'évent (26) et de l'élément de condensation (42), le second matériau (48) présentant une seconde résistance thermique, et dans lequel :
la première résistance thermique est plus élevée que la seconde résistance thermique pour condenser des vapeurs au niveau de l'élément de condensation (42) avant d'être libérées hors du dispositif par l'évent (26).

7. Procédé (500) pour déterminer des paramètres de transpiration d'un utilisateur, le procédé comprenant :
la collecte de la transpiration (510) provenant d'une première zone de peau (i) ;
la collecte d'un fluide (520) au niveau d'une seconde zone (ii) pour humidifier une structure microfluidique (10) d'un dispositif (1) présentant
un capteur (12) configuré pour déterminer un paramètre de transpiration à partir de la transpiration provenant d'une première zone de peau (i), dans lequel la structure microfluidique (10) comprend un premier absorbeur (20), relié par le biais d'un canal microfluidique (22) à la chambre de collecte ( 16) et au capteur (12), dans lequel le premier absorbeur (20) est configuré pour recevoir de la transpiration en provenance de la première zone de peau (i), ayant traversé le capteur (12), et
une chambre de régulation de l'évaporation (14), en connexion fluidique avec la structure microfluidique (10), configurée pour utiliser un fluide collecté au niveau d'une seconde zone (ii) pour humidifier la structure microfluidique (10), dans lequel la seconde zone (ii) est
une seconde zone de peau, et/ou
une surface d'un élément de condensation (42) configurée pour condenser des vapeurs libérées par le dispositif W par un évent (26) relié à la structure microfluidique par le biais de l'élément de condensation (42), et le capteur (12) comprenant un échappement (21) configuré pour évacuer la transpiration hors du capteur (12) et jusque dans la structure microfluidique (10), et
dans lequel la chambre de régulation de l'évaporation (14) est en connexion directe avec le premier absorbeur (20) et est configurée pour humidifier le premier absorbeur, et
la détermination d'un paramètre de transpiration (530) à partir de la transpiration provenant de la première zone de peau (i) à l'aide du capteur (12).
